(19) Europäisches Patentamt
European Patent Office
Office européen des brevets

(11) **EP 4 184 525 A1**

(12) **DEMANDE DE BREVET EUROPEEN**

(43) Date de publication:
**24.05.2023 Bulletin 2023/21**

(21) Numéro de dépôt: **22208493.1**

(22) Date de dépôt: **21.11.2022**

(51) Classification Internationale des Brevets (IPC):
**G16H 50/20** (2018.01) **G16H 50/30** (2018.01)
**G16H 10/60** (2018.01) **G16H 50/70** (2018.01)

(52) Classification Coopérative des Brevets (CPC):
**G16H 50/20; G16H 50/30;** G16H 10/60;
G16H 50/70

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**KH MA MD TN**

(30) Priorité: **22.11.2021 FR 2112345**

(71) Demandeur: **Commissariat à l'énergie atomique et aux énergies alternatives**
**75015 Paris (FR)**

(72) Inventeur: **BERDOUZ QRICHI ANIBA, Hakima**
**75014 PARIS (FR)**

(74) Mandataire: **Lavoix**
**2, place d'Estienne d'Orves**
**75441 Paris Cedex 09 (FR)**

(54) **PROCÉDÉ ET SYSTÈME DE DÉTECTION ET DE CARACTÉRISATION DE SIGNAUX FAIBLES D'EXPOSITION À UN RISQUE POUR UN PATIENT**

(57) - L'invention concerne un procédé et un système de détection et de caractérisation de signaux faibles d'exposition à un risque pour un patient, un signal faible étant représentatif d'une incubation d'une pathologie, à partir de données relatives au patient collectées sur un intervalle temporel donné. Le système comporte un module (36) de calcul d'une signature prédictive du risque, un module (38) de détection de présence d'au moins un signal faible d'exposition à un risque par comparaison de la signature prédictive du risque calculée à des signatures de risque de référence prédéterminées, et en cas de détection positive, application d'un module (40) de détermination d'une signature prédictive de référence associée à la signature prédictive du risque calculée et de caractérisation du risque associé à la signature de risque de référence, comportant un module affichage d'un scénario de menaces préalablement déterminé et enregistré en association avec ladite signature prédictive de référence.

FIG.1

EP 4 184 525 A1

**Description**

La présente invention concerne un procédé et un système de détection et de caractérisation de signaux faibles d'exposition à un risque pour un patient.

L'invention se situe dans le domaine de la santé numérique, et plus particulièrement de la détection d'exposition d'un patient à un risque clinique, pathologique et/ou thérapeutique, et trouve notamment des applications dans le dépistage précoce, le diagnostic assisté et la surveillance thérapeutique de maladies rares et/ou de polypathologies complexes et/ou systémiques.

Le terme « polypathologies » est employé lorsque le patient est atteint de plusieurs affections caractérisées, entraînant un état pathologique invalidant et nécessitant des soins continus d'une durée prévisible supérieure à six mois.

Dans le contexte de l'invention, on définit un signal faible d'exposition à un risque d'un patient, comme une information d'alerte, une irrégularité observée de faible intensité, annonciatrice d'une probabilité significative d'altération de l'état de santé du patient, associée à une pathologie complexe, à une évolution silencieuse par exemple de l'activité de la maladie, ou d'une incubation de nouveaux risques émergents auxquels le patient pourrait avoir été exposé, le long de son parcours de soins par exemple et/ou pendant sa prise en charge.

Dans le contexte de l'invention, le risque désigne un risque pathologique, c'est-à-dire associé au développement d'une pathologie complexe, ou un risque thérapeutique ou bien un risque clinique.

Un risque est la possibilité qu'un événement redouté survienne et que ses effets impactent l'état de santé du patient, sa prise en charge et sa qualité de vie.

La vraisemblance d'un risque est l'estimation de la faisabilité ou de la probabilité qu'un risque se réalise, selon l'échelle adoptée (très faible, peu vraisemblable, quasi certain, etc).

On entend par risque thérapeutique un risque associé à une thérapie administrée à un patient, par exemple un risque de développer des effets secondaires, événements indésirables ou un risque d'inefficacité d'une thérapie donnée et/ou de pharmaco-résistance.

Un risque clinique est un risque associé à une hospitalisation et/ou à une prise en charge médicale, comme par exemple un risque transfusionnel ou risque infectieux, gestion de l'identification du patient, risque médicamenteux, iatrogénie associée aux actes techniques, etc).

Un risque a une signature de risque associée, qui est une fonction mathématique modélisant l'exposition au risque dans le temps. Il est possible de calculer un score du risque, qui caractérise une quantification du risque à un instant temporel donné. Le score est la valeur à l'instant t de la signature du risque.

L'invention a notamment pour but d'automatiser le diagnostic prédictif et la surveillance thérapeutique, de manière à fournir une aide à la prise de décision clinique et thérapeutique, informée du risque.

Dans ce domaine, la détection et la caractérisation précoce de l'exposition d'un patient à un risque est critique pour permettre une prise en charge précoce et coordonnée, augmenter les chances de traitement efficace pour le patient et améliorer son état de santé et sa qualité de vie.

La question de la captation et de la caractérisation de signaux faibles, précurseurs d'un événement important ou critique (également appelé « évènement redouté »), se pose de manière plus générale, outre le domaine de la santé, dans le domaine industriel ou dans le domaine de la surveillance de risque de catastrophes naturelles.

La notion de signaux faibles, précurseurs à l'incubation d'événements redoutés, a été définie plus généralement, en particulier dans les sciences sociales. Il a été démontré *a posteriori* que toute défaillance aurait un signal précurseur nommé signal faible.

Une principale difficulté est la détection *a priori* et la caractérisation précoce de tels signaux faibles par rapport à des signaux aléatoires, qui sont qualifiés de bruits, les signaux faibles observables étant généralement eux-mêmes bruités dans une certaine mesure.

Diverses approches de détection et de caractérisation de signaux faibles ont été mises en oeuvre, notamment une approche symbolique et une approche numérique. L'approche symbolique s'appuie sur des modèles et des systèmes de raisonnement à base de règles, tentant de reproduire les mécanismes cognitifs d'un expert. Cette approche est limitée à des cas particuliers. L'approche numérique utilise des réseaux de neurones artificiels appliqués sur des données numériques, fondés sur des apprentissages automatiques. Cette approche peut s'avérer complexe, et permet notamment de réaliser des fouilles de données *a posteriori,* après l'occurrence d'un événement redouté. La détection en amont et *a priori* de signaux faibles demeure problématique.

Le brevet FR 3009615 décrit un procédé et un système pour capter et caractériser des signaux faibles comparés à une valeur seuil donnée, un signal étant associé à une quantité d'énergie et émis par une ou plusieurs sources à surveiller au sein d'un système. Ce procédé met en œuvre un calcul d'une signature traduisant une valeur d'énergie d'un signal détecté associé à un événement, en fonction d'une gravité G d'un événement ayant impacté la source émettrice du signal, une probabilité d'occurrence de l'événement et une fonction $M_R$ représentant la maîtrise du risque. La fonction de maîtrise du risque est pondérée par des paramètres représentant des moyens, des compétences et des méthodes déployées en prévention. Ce procédé permet de détecter des signaux faibles précurseurs d'un événement important (par exemple un dysfonctionnement) par comparaison à des seuils donnés.

L'invention a pour objet de proposer une méthode de caractérisation des signaux faibles améliorée par rapport à cette méthode de l'état de la technique, applicable dans le domaine de la santé et permettant de caractériser en particulier l'incubation d'une pathologie ou de poussées silencieuses (reprise de l'activité d'une pathologie) et/ou de l'altération de l'état de santé du patient.

A cet effet, l'invention propose, selon un aspect, un procédé de détection et de caractérisation de signaux faibles d'exposition à un risque pour un patient, un signal faible étant représentatif d'une incubation d'une pathologie, à partir de données relatives au patient collectées sur un intervalle temporel donné. Ce procédé étant caractérisé en ce qu'il comporte les étapes suivantes, mises en œuvre par un processeur :

- à partir de données relatives au patient collectées pendant l'intervalle temporel, calcul d'une signature prédictive du risque, la signature prédictive du risque comprenant un premier terme obtenu par sommation de signatures élémentaires associées à des évènements initiateurs élémentaires, une signature élémentaire étant dépendante de paramètres comprenant une valeur de gravité de l'événement initiateur élémentaire, une fonction caractéristique de l'événement initiateur élémentaire et une fonction de pondération associée à l'événement initiateur élémentaire, au moins une partie desdits paramètres étant déterminés par mise en œuvre d'un réseau de neurones,
- détection de présence d'au moins un signal faible d'exposition à un risque par comparaison de la signature prédictive du risque calculée à des signatures de risque de référence prédéterminées,
- en cas de détection positive, détermination d'une signature prédictive de référence associée à la signature prédictive du risque calculée et caractérisation du risque associé à ladite signature de risque de référence, ladite caractérisation comportant un affichage d'un scénario de menaces préalablement déterminé et enregistré en association avec ladite signature prédictive de référence.

Avantageusement, le procédé de détection et de caractérisation de signaux faibles d'exposition à un risque pour un patient met en œuvre une signature prédictive du risque, calculée en fonction d'événements initiateurs élémentaires, en prenant en compte des paramètres déterminés par mise en oeuvre de méthodes d'intelligence artificielle.

Avantageusement, l'approche proposée est une approche multi-modale combinant l'approche symbolique et l'approche numérique.

Le procédé de détection et de caractérisation de signaux faibles d'exposition à un risque pour un patient selon l'invention peut également présenter une ou plusieurs des caractéristiques ci-dessous, prises indépendamment ou selon toutes les combinaisons techniquement envisageables.

La fonction de pondération associée à l'évènement initiateur élémentaire est une fonction déterministe-probabiliste, dépendante d'une probabilité dudit événement initiateur élémentaire en lien avec l'incubation de ladite pathologie.

La signature prédictive du risque comporte un deuxième terme dépendant de paires d'événements initiateurs élémentaires et d'une fonction caractéristique d'intercorrélation pour chaque paire d'événements initiateurs élémentaires.

Le calcul d'une signature de risque prend en compte en outre une fonction probabiliste caractéristique de bruit relatif aux données collectées.

La signature élémentaire d'un événement initiateur élémentaire $E_i$ est fournie par la formule suivante :

$$2^{G_i} w_i(t) \sigma_i(t)$$

où Gi est la gravité de l'évènement initiateur élémentaire $E_i$, $\sigma_i(t)$ est la fonction caractéristique de l'événement initiateur élémentaire $E_i$ et $w_i(t)$ est la fonction de pondération associée à l'événement initiateur élémentaire $E_i$.

La gravité d'un événement initiateur élémentaire prend quatre valeurs différentes représentatives respectivement d'une gravité nulle, d'une gravité mineure, d'une gravité significative ou d'une gravité sévère.

La signature prédictive du risque est calculée selon la formule :

$$\Gamma(t) = \left[ \sum_i 2^{G_i} w_i(t) \sigma_i(t) + \sum_{jk} \xi_{jk} w_j(t) w_k(t) 2^{G_j + G_k} \right] * B(t)$$

Où Gi est la gravité de l'évènement initiateur élémentaire $E_i$, $\sigma_i(t)$ est la fonction caractéristique de l'événement initiateur élémentaire $E_i$, $w_i(t)$ est la fonction de pondération associée à l'événement initiateur élémentaire $E_i$ ; $\xi_{jk}$ est une fonction caractéristique d'intercorrélation entre événements initiateurs élémentaires $E_j$ et $E_k$, et B(t) est une fonction probabiliste caractérisant un bruit.

L'étape de détection de présence d'au moins un signal faible d'exposition à un risque comprend en outre une évaluation statistique d'une incertitude associée à ladite détection.

Le procédé comporte, suite à la collecte de données relatives au patient pendant l'intervalle temporel, un prétraitement desdites données collectées pour formater lesdites données collectées en données numériques, et une classification par un classifieur desdites données numériques pour obtenir des valeurs de paramètres associés aux évènements initiateurs élémentaires.

Le procédé comporte une phase d'initialisation d'une base de données des signatures de risque de référence, en lien avec un périmètre pathologique défini, en fonction de données de santé de cohortes de patients et de validations d'experts, et une mémorisation des signatures de risque de référence, de scénarios de menaces associés et d'une cartographie de risques associée.

Selon un autre aspect, l'invention concerne un système de détection et de caractérisation de signaux faibles d'exposition à un risque pour un patient, un signal faible étant représentatif d'une incubation d'une pathologie, à partir de données relatives au patient collectées sur un intervalle temporel donné. Le système comporte au moins un système de calcul, comportant un processeur configuré pour mettre en oeuvre :

- à partir de données relatives au patient collectées pendant l'intervalle temporel, un module de calcul d'une signature prédictive du risque, la signature prédictive du risque comprenant un premier terme obtenu par sommation de signatures élémentaires associées à des évènements initiateurs élémentaires, une signature élémentaire étant dépendante de paramètres comprenant une valeur de gravité de l'événement initiateur élémentaire, une fonction caractéristique de l'événement initiateur élémentaire et une fonction de pondération associée à l'événement initiateur élémentaire, au moins une partie desdits paramètres étant déterminés par mise en oeuvre d'un réseau de neurones,
- un module de détection de présence d'au moins un signal faible d'exposition à un risque par comparaison de la signature prédictive du risque calculée à des signatures de risque de référence prédéterminées,

en cas de détection positive, application d'un module de détermination d'une signature prédictive de référence associée à la signature prédictive du risque calculée et de caractérisation du risque associé à la signature de risque de référence, comportant un module affichage d'un scénario de menaces préalablement déterminé et enregistré en association avec ladite signature prédictive de référence..

Le système est configuré pour mettre en oeuvre le procédé de détection et de caractérisation de signaux faibles d'exposition à un risque pour un patient, selon ses diverses variantes rappelées brièvement ci-dessus.

Selon un autre aspect, l'invention concerne un programme d'ordinateur comportant des instructions logicielles qui, lorsqu'elles sont mises en oeuvre par un dispositif électronique programmable, mettent en oeuvre un procédé de détection et de caractérisation de signaux faibles d'exposition à un risque pour un patient tel que brièvement décrit ci-dessus.

D'autres caractéristiques et avantages de l'invention ressortiront de la description qui en est donnée ci-dessous, à titre indicatif et nullement limitatif, en référence aux figures annexées, parmi lesquelles :

[Fig 1] la figure 1 est un synoptique d'un dispositif de détection et de caractérisation de signaux faibles d'exposition à un risque pour un patient selon un mode de réalisation;

[Fig 2] la figure 2 est un synoptique des principales étapes d'une phase d'initialisation d'un procédé détection et de caractérisation de signaux faibles d'exposition à un risque pour un patient, selon un mode de réalisation ;

[Fig 3] la figure 3 est un synoptique des principales étapes d'une phase de calcul de signature prédictive de risque dans un procédé de détection et de caractérisation de signaux faibles d'exposition à un risque pour un patient, dans un mode de réalisation ;

[Fig 4] la figure 4 est un synoptique des principales étapes d'une phase de caractérisation de signaux faibles d'exposition à un risque pour un patient dans un mode de réalisation.

L'invention sera décrite ci-après dans des modes de réalisation, en particulier dans son application pour la détection et la caractérisation de signaux faibles d'exposition d'un patient à un risque de Lupus systémique.

Bien entendu, il s'agit d'un exemple non limitatif d'application de l'invention.

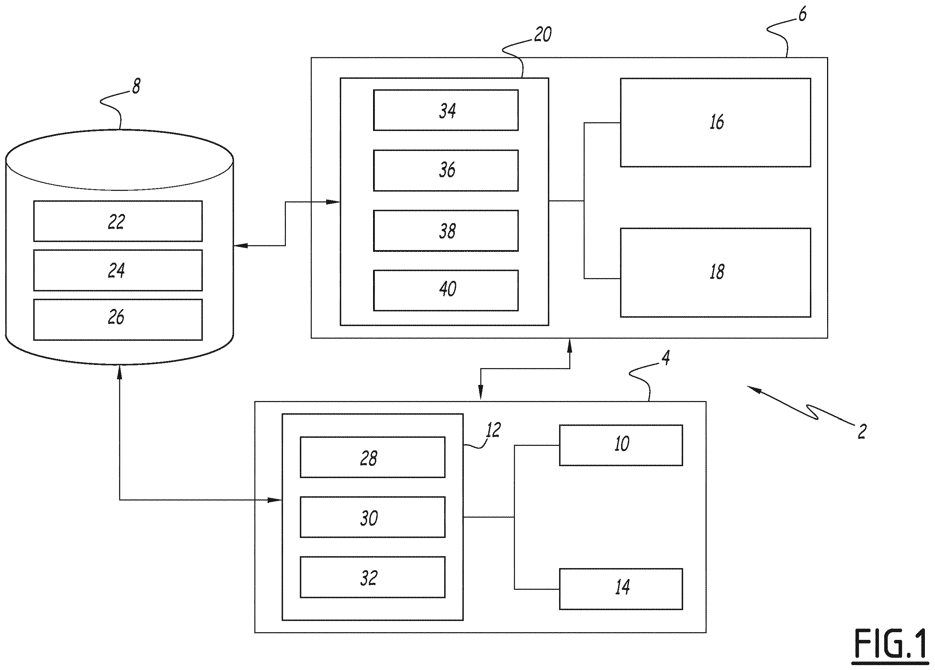

La **figure 1** illustre schématiquement un système 2 de détection et de caractérisation de signaux faibles d'exposition d'un patient à un risque.

Ce système 2 comprend un premier système de calcul 4 et un deuxième système de calcul 6. Dans un mode de réalisation, chacun des systèmes de calcul 4, 6 est formé par un ou plusieurs dispositifs électroniques programmables, e.g. ordinateurs, adaptés pour effectuer des calculs.

Ces systèmes de calcul 4, 6 sont adaptés pour communiquer, en lecture et en écriture, avec un système de stockage de données 8, qui comprend des bases de données mémorisées sur une ou plusieurs unités de mémoire électroniques.

Le premier système de calcul 4 comporte une unité de calcul 10, composée d'un ou plusieurs processeurs, associée à une unité de mémoire électronique 12 et à une interface homme-machine 14.

Le deuxième système de calcul 6 comporte une unité de calcul 16, composée d'un ou plusieurs processeurs, associée à une unité de mémoire électronique 20 et à une interface homme-machine 18.

Le premier système de calcul 4 est configuré pour mettre en oeuvre une phase d'initialisation d'un procédé de détection et de caractérisation de signaux faibles d'exposition d'un patient à un risque, en lien avec un périmètre pathologique prédéfini, permettant de générer ou d'enrichir des bases de données comprenant :

- une base de données 22 d'événements initiateurs élémentaires et de paramètres associés caractérisant des risques pour le périmètre pathologique prédéfini ;
- une base 24 de signatures de risques de référence et de scénarios de menaces associés ;
- une cartographie de risques associée 26 est optionnellement mémorisée.

On entend ici par scénario de menaces associées à un risque, également appelé scénario de risque, un scénario complet d'évolution allant de la source du risque, e.g. un ou plusieurs évènements initiateurs élémentaires à son développement.

Un évènement initiateur élémentaire est caractérisé par un ou plusieurs paramètres sortant d'un intervalle de valeurs nominales, représentatif d'un signal faible précurseur du risque. Il s'agit par exemple d'un symptôme du patient, ou d'un biomarqueur du patient.

Par exemple un scénario menaces associé au risque décrit des évolutions temporelles d'une pathologie, par exemple par des évolutions de symptômes du patient. En d'autres termes, un scénario de menaces est un modèle cinétique de la pathologie, également appelé « modèle mécanistique ».

Une cartographie associée est une représentation visuelle, par exemple sous forme de diagramme 2D ou 3D, des risques pouvant affecter l'état de santé d'un patient.

Ces bases de données 22, 24, 26 sont mémorisées par le système de stockage de données 8. Le système de stockage de données est un support lisible par ordinateur est par exemple, un médium apte à mémoriser les instructions électroniques et à être couplé à un bus d'un système informatique. A titre d'exemple, le support lisible est un disque optique, un disque magnéto-optique, une mémoire ROM, une mémoire RAM, tout type de mémoire non-volatile (par exemple EPROM, EEPROM, FLASH, NVRAM), une carte magnétique ou une carte optique.

L'unité de calcul 10 configurée(s) pour mettre en oeuvre un module 28 de sélection et validation de modèles de risque associé au périmètre, un module 30 de calcul de signatures de risque de référence, de scénarios de menaces associés et de cartographie de risque associée et un module 32 de validation de mise à jour. Chaque risque est modélisé par un modèle multi-physique en fonction de données récoltées sur une ou plusieurs cohortes de patients sur une durée, ce modèle pouvant être mis à jour au fur et à mesure en fonction de chaque patient, comme expliqué plus en détail ci-après.

Dans un mode de réalisation, pour un périmètre pathologique dit global, plusieurs risques sont pris en considération, chaque risque ayant un modèle de risque associé, et un modèle de risque global, prenant en compte les interdépendances et les corrélations entre les risques, est obtenu.

Le deuxième système de calcul 6 est configuré pour mettre en oeuvre un procédé de détection et de caractérisation de signaux faibles d'exposition à un risque pour un patient donné.

L'unité de calcul 16 est configurée pour mettre en œuvre :

- un module 34 de collecte de données relatives au patient pendant un intervalle temporel donné, le module 34 étant configuré pour recevoir des données numériques collectées représentatives notamment de mesures physiologiques relatives au patient obtenues préalablement et enregistrées ;
- un module 36 de calcul d'une signature prédictive de risque ;
- un module 38 de détection de présence d'au moins un signal faible d'exposition à un risque par comparaison de la signature prédictive du risque à des signatures de risque de référence ;
- un module 40 de détermination d'une signature prédictive de référence associée à la signature prédictive du risque calculée et caractérisation de risque associé à la signature de risque de référence, ce module comportant également un module d'affichage de données sur l'interface homme-machine 18, notamment sur un écran d'affichage de cette interface.

Dans un mode de réalisation, les modules 34, 36, 38, 40 sont réalisés sous forme de code logiciel, et forment un programme d'ordinateur, comportant des instructions logicielles qui, lorsqu'elles sont mises en oeuvre par un dispositif électronique programmable, mettent en oeuvre un procédé de détection et de caractérisation de signaux faibles d'exposition à un risque.

En variante non représentée, les modules 34, 36, 38, 40 sont réalisés chacun sous forme d'un composant logique programmable, tel qu'un FPGA (de l'anglais *Field Programmable Gate Array*), ou un GPGPU (de l'anglais *General-purpose processing on graphics processing),* ou encore sous forme d'un circuit intégré dédié, tel qu'un ASIC (de l'anglais *Application Specific Integrated Circuit).*

Le programme d'ordinateur de détection et de caractérisation de signaux faibles d'exposition à un risque est en outre apte à être enregistré sur un support, non représenté, lisible par ordinateur. Le support lisible par ordinateur est par exemple, un médium apte à mémoriser les instructions électroniques et à être couplé à un bus d'un système informatique. A titre d'exemple, le support lisible est un disque optique, un disque magnéto-optique, une mémoire ROM, une mémoire RAM, tout type de mémoire non-volatile (par exemple EPROM, EEPROM, FLASH, NVRAM), une carte magnétique ou une carte optique.

De manière analogue, les modules 28, 30, 32 sont réalisés sous forme de code logiciel, et forment un programme d'ordinateur. En variante non représentée, les modules 28, 30, 32 sont réalisés chacun sous forme d'un composant logique programmable, tel qu'un FPGA (de l'anglais *Field Programmable Gate Array),* ou un GPGPU (de l'anglais *General-purpose processing on graphies processing),* ou encore sous forme d'un circuit intégré dédié, tel qu'un ASIC (de l'anglais *Application Specific Integrated Circuit).*

Le premier système de calcul 4 et le deuxième système de calcul 6 ont été représentés ici sous forme de systèmes de calcul distincts.

Dans une variante non représentée, les deux systèmes de calcul 4, 6 sont réunis en un seul système de calcul, qui effectue à la fois la phase d'initialisation pour un périmètre pathologique défini et la phase de traitement des données d'un patient pour la caractérisation et la prédiction de signaux faibles d'exposition à un risque pour un patient.

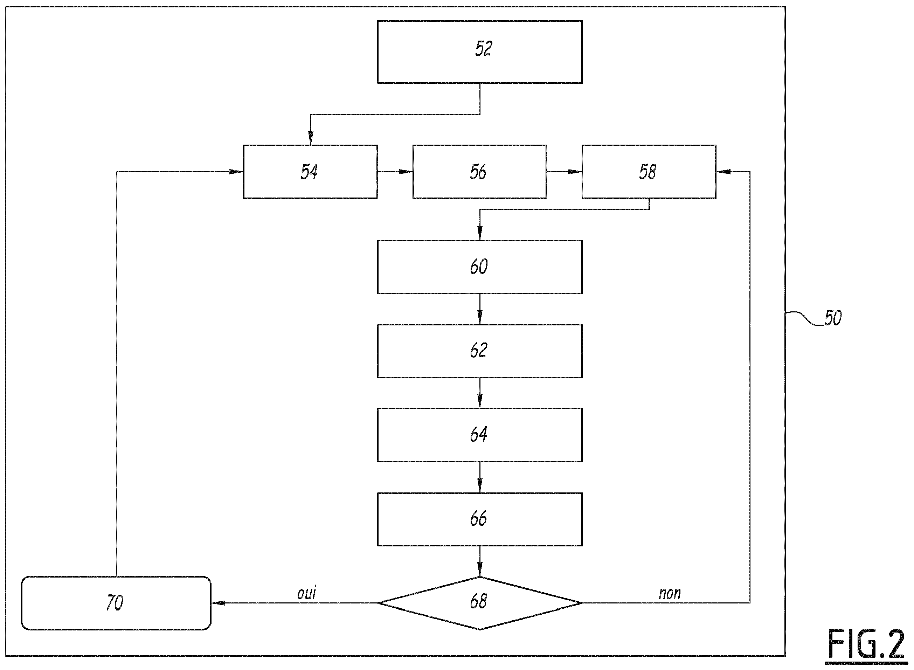

La **figure** 2 est un synoptique des principales étapes d'une phase d'initialisation 50 d'un procédé de détection et de caractérisation de signaux faibles d'exposition à un risque, dans un mode de réalisation.

Cette phase d'initialisation est une phase préalable à la mise en oeuvre du procédé pour un patient donné, et elle a pour objectif de générer et mémoriser les informations :

- de la base de données 22 d'événements initiateurs élémentaires et de paramètres associés caractérisant des risques pour le périmètre pathologique prédéfini ;
- de la base 24 de signatures de risques de référence et de scénarios de menaces associés ;
- de la cartographie de risques associée 26.

Avantageusement, la phase d'initialisation est effectuée, en lien avec un périmètre pathologique, en fonction de données de santé de cohortes de patients et de validations d'experts. Par exemple, la phase d'initialisation 50 est menée par un expert qui est un professionnel de santé.

Par exemple, les données de santé de cohortes de patients sont obtenues d'un système de stockage distant. Ces données permettent d'obtenir des statistiques collectives.

Dans un mode de réalisation, la phase d'initialisation est menée par un expert, par exemple un professionnel de santé, qui utilise une interface homme-machine (e.g. écran et clavier, écran tactile, interface de commande vocale...) lui permettant de sélectionner lors d'une étape 52 un périmètre pathologique à investiguer. Par exemple, le périmètre pathologique à investiguer est une pathologie affectant plusieurs organes, comme par exemple le lupus systémique.

Selon une variante, le périmètre pathologique à investiguer est en lien avec un organe ou un sous-ensembles d'organes (coeur, rein, poumon...).

Le procédé comprend ensuite une sélection 54 de données de santé de cohortes de patients, par exemple préalablement mémorisées dans une ou plusieurs bases de données, atteints de la pathologie à investiguer ou atteints de pathologies en lien avec le ou les organes à investiguer. En complément optionnel, des données, par exemple sous forme de documents, articles, littérature scientifique, en lien avec le périmètre pathologique à investiguer sont également obtenus.

De plus, l'expert a la possibilité de sélectionner à l'étape 56 des modèles et algorithmes d'apprentissage par intelligence artificielle, à déployer dans le procédé, parmi plusieurs tels modèles et algorithmes proposés, par exemple à partir d'évaluations de performance issues d'un retour d'expérience opérationnel ou de la littérature scientifique. Par exemple, il est possible d'utiliser des algorithmes d'apprentissage profond mettant en œuvre des réseaux de neurones artificiels, de manière automatisée, parmi :

- apprentissage supervisé basé sur des réseaux de neurones convolutifs (CNN), comprenant plusieurs couches, qui sont, en option, entièrement connectées ;
- apprentissage semi-supervisé basé par exemple sur des réseaux de neurones profonds (DNN) ;
- apprentissage non-supervisé basé par exemple sur des réseaux de neurones récurrents (RNN) à mémoire longue et courte (ou LSTM pour « long short-term memory » en anglais), comprenant une ou plusieurs couches LSTM.

Le procédé comprend également une étape 58 d'obtention de modèles multi-physiques du risque s'ils existent pour la pathologie à investiguer.

On appelle ici modèle multi-physique du risque un modèle intégrant plusieurs paramètres permettant de caractériser le risque, par exemple des paramètres physiologiques du patient, des biomarqueurs, des symptômes qui sont quantifiables.

Un tel modèle définit des évènements initiateurs élémentaires du risque dont l'utilisation est décrite plus en détail ci-après.

L'étape d'obtention 58 est par exemple mise en oeuvre par mise en oeuvre d'un algorithme d'intelligence artificielle parmi les algorithmes mentionnés ci-dessus, entraîné dans la phase d'apprentissage sur les données collectées à l'étape de sélection 54.

Selon un mode de réalisation, l'étape d'obtention 58 effectue une sélection parmi des modèles fournis par des experts, la sélection étant par exemple effectuée sur un critère de performance choisi.

Selon une variante, l'étape d'obtention 58 effectue une construction d'un modèle de risque à partir des données collectées à l'étape 54.

Le procédé comprend également, de préférence, une étape 60 de validation par interaction avec l'expert, permettant une validation incrémentale des résultats intermédiaires, permettant par exemple d'affiner et de renforcer l'apprentissage. Par exemple, dans un mode de réalisation, l'étape 60 est réalisée par un module de questions/réponses (ou QA de « questions and answers » en anglais), par exemple mis en oeuvre sous forme d'un agent conversationnel (ou « chatbot » en anglais). Une telle étape 60 de validation par interaction fait partie d'un processus de type HILL (pour « human in the loop learning » en anglais), qui permet d'améliorer les résultats obtenus automatiquement par un apprentissage machine.

Le procédé comprend également une étape 62 de couplage multi-échelle de modèles multi-physiques du risque et des incertitudes associées, permettant d'obtenir des paramètres associés à des évènements initiateurs élémentaires, permettant de calculer une signature prédictive de risque, formée à partir signatures dites signatures élémentaires d'événements initiateurs élémentaires, comme détaillé ci-dessous.

Le couplage multi-échelle est mis en oeuvre par le modèle d'intelligence artificielle sélectionné à l'étape de sélection 56.

On entend ici par couplage multi-échelle, par exemple dans le cas d'un périmètre pathologique global impliquant plusieurs organes, la prise en considération des modèles de risque calculés pour chaque organe.

On entend ici par incertitude associée au modèle est une incertitude probabiliste, calculée par une méthode de calcul probabiliste par rapport aux données collectées.

En effet, les données collectées sont généralement biaisées voire bruitées à la source compte-tenu des incertitudes associées aux systèmes et procédés d'acquisition des données à la source, de leur traitement et de leur sauvegarde. Il peut s'agir de données manquantes à l'acquisition voire des données erronées lors de la saisie et/ou l'interprétation par le clinicien ou l'opérateur. Les modèles mathématiques utilisés génèrent également des incertitudes supplémentaires liées aux écarts entre le modèle réel décrivant la mécanistique et la phénoménologie de l'exposition aux risques et les approximations déployées en fonction des données disponibles.

Pour évaluer cette incertitude, plusieurs méthodes sont décrites par l'état de l'art. En témoigne, l'utilisation d'une loi de probabilité, comme la loi de Poisson qui s'applique à l'apparition d'événements de faibles probabilité ou bien la loi de Gauss (ou loi normale) qui est la loi de probabilité la plus utilisée. Son intérêt est confirmé si les conditions suivantes sont réalisées simultanément :

- Les causes d'erreur sont nombreuses ;
- Les erreurs sont du même ordre de grandeur ;
  Les fluctuations liées aux différentes causes d'erreur sont indépendantes et additives.

Le procédé comprend en outre une étape 64 de calcul de cartographie du risque et de modélisation déterministe-probabiliste du risque et des menaces associées.

La modélisation déterministe-probabiliste comprend la prise en compte de paramètres déterministes (par exemple âge du patient, sexe du patient etc), qui modifient les calculs d'incertitude probabiliste associées au risque. Par exemple, une pathologie a une prévalence supérieure dans certaines classes d'âge, ou chez les hommes etc.

Pour un risque considéré, on applique une classification par réseaux de neurones ou par forêts aléatoires en plusieurs classes en fonction de la modélisation déterministe-probabiliste et pour chaque classe, on calcule une signature de risque de référence qui est mémorisée dans la base de données 24, ainsi qu'un scénario de menaces associé.

Pour illustrer un scénario de menaces et de cartographie du risque dans le cas du Lupus systémique, prenons le cas d'une jeune patiente atteinte de Lupus systémique, portant un projet de grossesse. Cette situation est très fréquente dès lors que 90% des patients lupiques sont des patientes, jeunes et en âge de procréer en majorité (âgées entre 20 et 40 ans). Pour engager un projet de grossesse, l'activité de la maladie devrait être stabilisée pendant au moins de 18 mois. Dans ce cadre, le scénario de menaces pourrait être l'apparition de micro-poussées et/ou d'atteintes rénales, caractérisées par leur incubation silencieuse, pouvant compromettre la grossesse et la santé de la patiente et de son enfant, si elles ne sont pas détectées de manière précoce. La cartographie des risques est par conséquent, l'ensemble des risques associés à un projet de grossesse, qu'ils soient intrinsèques à la pathologie ou bien aux événements potentiels liés à la grossesse (diabète gestationnel...) ou aux traitements thérapeutiques long cours.

La signature élémentaire d'un évènement initiateur élémentaire $E_i$ est définie par la formule suivante :

[MATH 1]

$$Sig\_E_i(t) = 2^{G_i} w_i(t) \sigma_i(t)$$

Où $G_i$ est la gravité de l'évènement initiateur élémentaire $E_i$, $\sigma_i(t)$ est la fonction caractéristique de l'événement initiateur élémentaire $E_i$ et $w_i(t)$ est la fonction de pondération associée à l'événement initiateur élémentaire $E_i$.

La variable t représente le temps, les fonctions respectives pouvant être, dans des modes de réalisation, évolutives en fonction du temps.

Par exemple, la gravité est une fonction de l'événement initiateur élémentaire.

Dans un mode de réalisation, la gravité peut prendre quatre valeurs différentes représentatives respectivement d'une gravité nulle, d'une gravité mineure, d'une gravité significative ou d'une gravité sévère.

Par exemple, la gravité prend les valeurs suivantes : 0 pour gravité nulle, 1 pour gravité mineure, 2 pour gravité significative et 3 pour gravité sévère.

La fonction caractéristique d'un événement initiateur élémentaire $E_i$ prend par exemple les valeurs 0 ou 1, en fonction de l'état de réalisation de l'évènement :

$\sigma_i(t) = 1$ si $E_i$ s'est réalisé
$\sigma_i(t) = 0$ sinon

La fonction de pondération $w_i(t)$ est par exemple un paramètre fixé par un expert ou une fonction déterministe-probabiliste associée à un évènement initiateur élémentaire $E_i$, caractérisé par une gravité $G_i$, et une probabilité $p_i$. La fonction de pondération peut également dépendre du patient, par exemple si le patient a des facteurs de risques aggravant la pathologie en lien avec l'évènement initiateur élémentaire $E_i$, par exemple une exposition à des substances chimiques dont l'effet aggravant est connu.

Par exemple, une formule de la pondération est :

[MATH 2]

$$w_i = \prod_k V_{ik} * p_i$$

Où $V_{ik}$ est une valeur représentative d'un facteur de risque k du patient, en lien avec l'événement initiateur élémentaire $E_i$.

Dans un mode de réalisation, la signature prédictive de risque est calculée selon la formule suivante qui fournit $\Gamma(t)$, également appelée fonction d'incubation:

[MATH 3]

$$\Gamma(t) = \left[ \sum_i 2^{G_i} w_i(t) \sigma_i(t) + \sum_{jk} \xi_{jk} w_j(t) w_k(t) 2^{G_j + G_k} \right] * B(t)$$

Où $G_i$ est la gravité de l'évènement initiateur élémentaire $E_i$, $\sigma_i(t)$ est la fonction caractéristique de l'événement initiateur élémentaire $E_i$, $w_i(t)$ est la fonction de pondération associée à l'événement initiateur élémentaire $E_i$ ; $\xi_{jk}$ est une fonction caractéristique d'intercorrélation entre événements initiateurs élémentaires $E_j$ et $E_k$, et $B(t)$ est une fonction probabiliste caractérisant un bruit.

La variable t représente le temps, les fonctions respectives pouvant être, dans des modes de réalisation, évolutives en fonction du temps.

Par exemple :

$\xi_{jk}$ = 1 si la corrélation des évènements initiateurs élémentaires $E_j$ et $E_k$ apporte un effet négatif aggravant ;

$\xi_{jk}$ = 0 si la corrélation des évènements initiateurs élémentaires $E_j$ et $E_k$ n'apporte aucun effet, en d'autres termes est neutre;

$\xi_{jk}$ = -1 si la corrélation des évènements initiateurs élémentaires $E_j$ et $E_k$ apporte un effet positif protecteur.

Plus généralement, si la corrélation des évènements initiateurs élémentaires $E_j$ et $E_k$ apporte un effet négatif aggravant $\xi_{jk}$ prend une première valeur de corrélation, de préférence une valeur positive, si la corrélation des évènements initiateurs élémentaires $E_j$ et $E_k$ apporte un effet positif protecteur, $\xi_{jk}$ prend une deuxième valeur de corrélation, de préférence négative.

Le bruit B(t) peut être filtré grâce à l'implémentation de fonctions mathématiques connues, ce qui permet d'obtenir une fonction d'incubation filtrée :

[MATH 4]

$$\hat{\Gamma}(t) = \left[ \sum_i 2^{G_i} w_i(t) \sigma_i(t) + \sum_{jk} \xi_{jk} w_j(t) w_k(t) 2^{G_j + G_k} \right]$$

La variable t représente le temps, les fonctions respectives pouvant être, dans des modes de réalisation, évolutives en fonction du temps.

Par exemple dans le cas du lupus systémique, la table 1 ci-dessous présente un tableau d'événements initiateurs élémentaires, considérés indépendants (i.e. fonction caractéristique d'intercorrélation égale à 0 entre événements), et de paramètres associés. Les évènements initiateurs élémentaires sont dans cet exemple des symptômes listés comme pouvant être liés à une incubation d'une activité du lupus d'un patient atteint par cette maladie (cf article de C.Bombardier et al, Dérivation of SLEDAI : a disease activity index for lupus patients », Arthritis Rheum, 1992, 35, 630-640).

Dans cet exemple, la fonction caractéristique de chaque évènement initiateur élémentaire est égale à 1 si l'événement se produit et à 0 si l'événement ne se produit pas.

La première colonne de la table 1 indique les évènements initiateurs élémentaires, la deuxième colonne une valeur de gravité associée, la troisième colonne une valeur de pondération associée, la quatrième colonne une valeur de fonction caractéristique associée, la cinquième colonne la signature élémentaire $\hat{\Gamma}i$ calculée et la sixième colonne la valeur de score SLEDAI fournie dans l'article cité ci-dessus.

Comme on peut le constater, la signature élémentaire calculée est égale au score SLEDAI pour chaque évènement initiateur élémentaire, les valeurs de score SLEDAI étant validées par des experts.

[TABLE 1]

| Evènements initiateurs élémentaires supposés indépendants et caractéristiques associées | $G_j$ | $w_i$ | $\sigma_i$ | $\hat{\Gamma}_i$ | Score SLEDAI |
|---|---|---|---|---|---|
| **Convulsions** apparition récente, exclusion des causes métaboliques, infectieuses ou médicamenteuses) | 3 | 1 | 1 | 8 | **8** |
| **Psychose** (perturbation de l'activité normale en rapport avec une altération sévère de la perception de la réalité. Comprend : hallucinations, incohérence, appauvrissement du contenu de la pensée, raisonnement illogique, comportement bizarre, désorganisé ou catatonique. Exclusion d'une insuffisance rénale ou d'une cause médicamenteuse) | 3 | 1 | 1 | 8 | **8** |
| **Atteinte cérébrale** (altération des fonctions mentales avec troubles de l'orientation, de la mémoire ou autre d'apparition brutale et d'évolution fluctuante. Comprend : troubles de la conscience avec réduction des capacités de concentration, incapacité à rester attentif avec en plus 2 au moins des manifestations suivantes : troubles perceptifs, discours incohérent, insomnie ou somnolence diurne, augmentation ou diminution de l'activité psychomotrice) | 3 | 1 | 1 | 8 | **8** |

(suite)

| Evènements initiateurs élémentaires supposés indépendants et caractéristiques associées | $G_j$ | $w_i$ | $\sigma_i$ | $\widehat{\Gamma}_\iota$ | Score SLEDAI |
|---|---|---|---|---|---|
| **Troubles visuels** (atteinte rétinienne du lupus. Comprend : nodules dysoriques, hémorragies rétiniennes, exsudats séreux ou hémorragies choroïdiennes, névrite optique. Exclusion d'une cause hypertensive, infectieuse ou médicamenteuse | 3 | 1 | 1 | 8 | 8 |
| **Nerfs crâniens** (neuropathie sensitive ou motrice d'apparition récente touchant un nerf crânien) | 3 | 1 | 1 | 8 | **8** |
| **Céphalées** (céphalées sévères et persistantes, pouvant être migraineuses mais résistant aux antalgiques majeurs) | 3 | 1 | 1 | 8 | **8** |
| **AVC** (accident vasculaire cérébral d'apparition récente. Artériosclérose exclue) | 3 | 1 | 1 | 8 | **8** |
| **Vascularité** (ulcérations, gangrène, nodules digitaux douloureux, infarctus péri-unguéaux ou preuve histologique ou artériographie de vascularite) | 3 | 1 | 1 | 8 | **8** |
| **Arthrites** (plus de 2 articulations douloureuses avec des signes inflammatoires locaux: douleur, tuméfaction ou épanchement articulaire) | 2 | 1 | 1 | 4 | **4** |
| **Myosite** (douleur/faiblesse musculaire proximale associées à une élévation des CPK et/ou aldolases ou à des modifications électromyographiques ou à une biopsie montrant des signes de vascularite) | 2 | 1 | 1 | 4 | **4** |
| **Cylindres urinaires** (cylindres de globules rouges) | 2 | 1 | 1 | 4 | **4** |
| **Hématurie** (> 5 GR / champ en l'absence de lithiase, d'infection ou d'une autre cause) | 2 | 1 | 1 | 4 | **4** |
| **Protéinurie** (>0,5 g/24h. Apparition récente ou majoration récente de plus de 0,5g/24h) | 2 | 1 | 1 | 4 | **4** |
| **Pyurie** (> 5 GB/champ en l'absence d'infection) | 2 | 1 | 1 | 4 | **4** |
| **Eruption cutanée** (apparition ou récurrence d'une éruption inflammatoire) | 1 | 1 | 1 | 2 | **2** |
| **Alopécie** (apparition récente ou récidive d'une alopécie en plaque ou diffuse) | 1 | 1 | 1 | 2 | **2** |
| **Ulcères muqueux** (apparition récente ou récidive d'ulcérations orales ou nasales) | 1 | 1 | 1 | 2 | **2** |
| **Pleurésie** (douleur thoracique d'origine pleurale avec frottement ou épanchement ou épaississement pleural) | 1 | 1 | 1 | 2 | **2** |
| **Péricardite** (douleur péricardique avec au moins l'une des manifestations suivantes : frottement, épanchement ou confirmation électrographique ou échographique) | 1 | 1 | 1 | 2 | **2** |
| **Complément** (diminution du CH50, du C3 ou du C4 < à la normale inférieure du laboratoire) | 1 | 1 | 1 | 2 | **2** |
| **Anti-ADN** (positivité > à 25% par le test de Farr ou taux > à la normale du laboratoire) | 1 | 1 | 1 | 2 | **2** |

(suite)

| Evènements initiateurs élémentaires supposés indépendants et caractéristiques associées | $G_j$ | $w_i$ | $\sigma_i$ | $\widehat{\Gamma_\iota}$ | Score SLEDAI |
|---|---|---|---|---|---|
| **Fièvre**<br>(>38° en l'absence de cause infectieuse) | 0 | 1 | 1 | 1 | **1** |

Par exemple, dans le cas d'application au lupus systémique, les événements initiateurs élémentaires listés dans la table 1 sont issus d'études d'experts. La caractérisation des facteurs de pondération $w_i(t)$ s'effectue de préférence par apprentissage IA par renforcement pour accroître la précision et la personnalisation des événements initiateurs élémentaires.

Le procédé comprend, optionnellement, une autre étape 66 de validation interactive par un expert, analogue à l'étape 60 décrite ci-dessus.

L'expert valide notamment les résultats des étapes 62 et 64.

En cas de validation positive (réponse 'oui' au test 68), la base de données 22 d'événements initiateurs élémentaires et de paramètres associés caractérisant des risques pour le périmètre pathologique prédéfini, la base 24 de signatures de risques de référence et de scénarios de menaces associés et la cartographie de risques associée 26 sont mises à jour (étape 70) avec les résultats des étapes 62 et 64.

En cas de validation négative (réponse 'non' au test 68), le procédé retourne à l'étape 58 de sélection de modèles multi-physiques du risque, et les étapes 60 à 68 sont itérées.

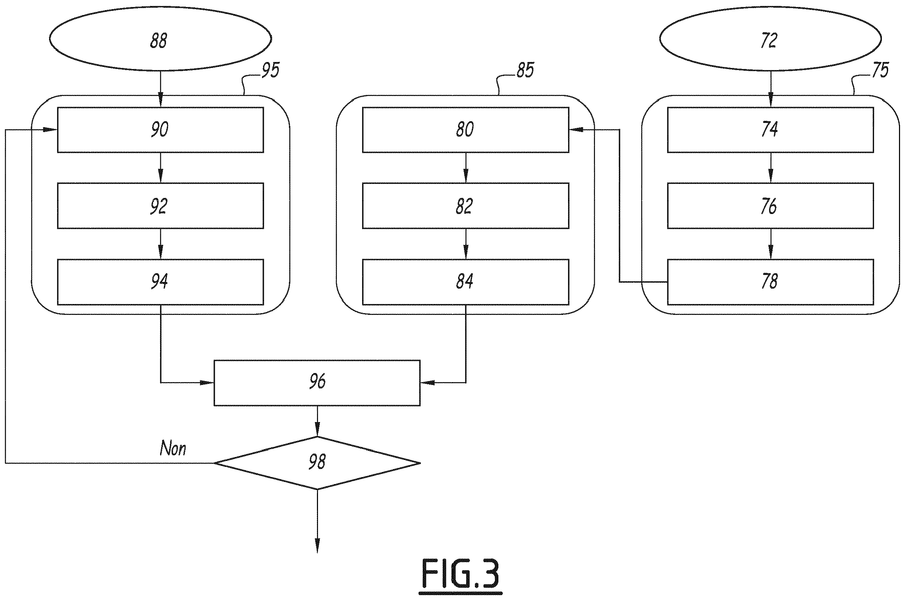

La **figure 3** est un synoptique des principales étapes d'une phase de calcul et d'évaluation de signature de risque dans le procédé de détection et de caractérisation de signaux faibles d'exposition à un risque pour un patient donné, dans un mode de réalisation.

Le procédé reçoit en entrée des données 72 relatives au patient sous forme numérique, comprenant des données physiologiques préalablement obtenues et enregistrées (par exemple résultats d'analyses médicales, température corporelle, rythme cardiaque, céphalées) et de diagnostic collectées pendant un intervalle temporel donné, dit intervalle temporel de surveillance, par exemple une semaine, 15 jours, un mois. Les données 72 relatives au patient peuvent également comprendre des données descriptives du patient (âge, sexe etc), des données historiques, par exemple des antécédents médicaux, et des données relatives à des facteurs de risque connus (par exemple exposition à des substances nocives, traitements médicamenteux).

Ces données 72 sont collectées lors d'une étape de collecte 74, par exemple sous forme de fichiers qui contiennent ces données et/ou par saisie par un opérateur. Ces données 72 collectées sont appelées données brutes.

La collecte de données est effectuée automatiquement par réception de données, par exemple à partir d'un dispositif porté par le patient, par exemple un dispositif de type montre connectée, comportant des capteurs permettant de mesurer des paramètres physiologiques, de les mémoriser et de les transmettre au deuxième système de calcul 6 par des moyens de transmission, ou à partir de données renseignées via une interface homme-machine d'un dispositif connecté configuré pour communiquer avec le deuxième système de calcul 6. Un tel dispositif connecté est par exemple un téléphone intelligent (ou smarphone), une tablette, un ordinateur.

Les données brutes sont prétraitées lors d'une étape de prétraitement numérique 76, ce prétraitement consistant à formater, ou en d'autres termes à structurer et à traduire, les données brutes en données numériques exploitables par la suite par des algorithmes de traitement automatiques. L'étape de prétraitement est réalisée par un traitement automatique sur la base de règles prédéterminé. Par exemple si le patient effectue un autotest dont le résultat est affiché par une bandelette de couleur, le patient renseigne la couleur du résultat, et le prétraitement 76 traite ce résultat en indiquant un intervalle de valeurs de biomarqueurs correspondant.

Ensuite le procédé comporte une étape 78 de classification des données numériques obtenues à l'étape 76 par une méthode d'intelligence artificielle. Par exemple l'étape 78 applique un classifieur, implémenté par un algorithme d'intelligence artificielle, tel un réseau de neurones, ou un arbre de décision ou un réseau de forêts, entraînés dans une phase d'apprentissage préalable.

En sortie de cette étape 78 de classification des données sont obtenues les paramètres définissant les évènements initiateurs élémentaires et les valeurs de gravité, de pondération associées.

Les évènements initiateurs élémentaires associés au risque que l'on cherche à caractériser sont définis par le modèle du risque calculé et mémorisé lors de la phase d'initialisation 50.

Les étapes 74, 76 et 78 contribuent à un prétraitement 75 des données 72 relatives au patient qui sont collectées.

Ce prétraitement 75 est suivi d'une évaluation prédictive 85 de l'incubation d'une pathologie définie par le périmètre pathologique prédéfini.

Cette évaluation prédictive comprend un calcul 80 de signature prédictive du risque redouté en utilisant la formule [MATH 3] ou [MATH 4] dans un mode de réalisation.

Le procédé comporte ensuite une étape 82 d'évaluation statistique d'incertitudes associées à la signature prédictive du risque calculée, cette évaluation prenant en compte des incertitudes associées aux données, modèles et algorithmes.

Cette évaluation statistique d'incertitudes est effectuée par une méthode de calcul statistique, par exemple en implémentant une loi normale ou bien une loi de Poisson selon une des méthodes connues dans l'état de l'art.

Le procédé comprend en outre une étape 84 d'évaluation temporelle de la fonction d'incubation ou signature prédictive du risque, selon la formule [MATH 3] ou [MATH 4], sur l'intervalle temporel de surveillance, avec une fréquence temporelle choisie. Ainsi, un échantillonnage temporel de la fonction d'incubation prédictive du risque (ou signature prédictive du risque) est obtenu, sur une durée temporelle donnée, formant une courbe d'évolution du risque. La durée temporelle est par exemple d'une ou plusieurs semaines ou mois.

Dans le mode de réalisation décrit, sensiblement en parallèle de l'évaluation prédictive 85 de l'incubation d'une pathologie pour la patient considéré, est mise en oeuvre une évaluation parallèle 95 à partir de données 88 mémorisées, également appelées données de retour d'expérience.

L'évaluation 95 effectuée à partir a pour objectif de permettre une mise à jour interactive des modèles mémorisés dans les bases de données 22, 24 en fonction des données collectées pour chaque patient, permettant ainsi d'affiner les modèles de risque, les signatures de risque de référence et des scénarios de menaces associés.

En plus, cette évaluation permet de mettre en lumière des scénarios rares, mais néanmoins possibles, ayant une très faible probabilité d'occurrence mais correspondant à un scénario redouté pour le patient.

L'évaluation 95 comprend une étape 90 d'obtention d'une signature de risque de référence et de modèle mécanistique du risque associé pour le patient donné. La signature de risque de référence est la plus proche du modèle calculé pour le patient donné, à partir des données 88 mémorisées, notamment à partir des bases de données 22, 24.

Ensuite, lors d'une étape 92, une prédiction de l'évolution du risque pour le patient est calculée, sur la même durée temporelle que celle utilisée à l'étape 84, en utilisant la signature du risque de référence obtenue à l'étape 90.

Une étape 94 d'évaluation déterministe-probabiliste de la signature du risque de référence appliquée et du scénario de menaces redouté associé est mise en oeuvre par des méthodes mathématiques du plus proche voisin par exemple

Une étape 96 de validation par interaction avec un expert, faisant partie d'un processus de type HILL (pour « human in the loop learning » en anglais), est ensuite mise en oeuvre, et si le résultat de la validation (test 98) est négatif, une modification de la signature de risque de référence dans la base de données est appliquée, par apprentissage par renforcement et les étapes 90, 92 et 94 sont itérées.

La validation comprend notamment la comparaison entre la signature de risque de référence et la signature de risque obtenue pour le patient.

L'expert valide alors les données de référence mémorisées dans les bases de données 22, 24.

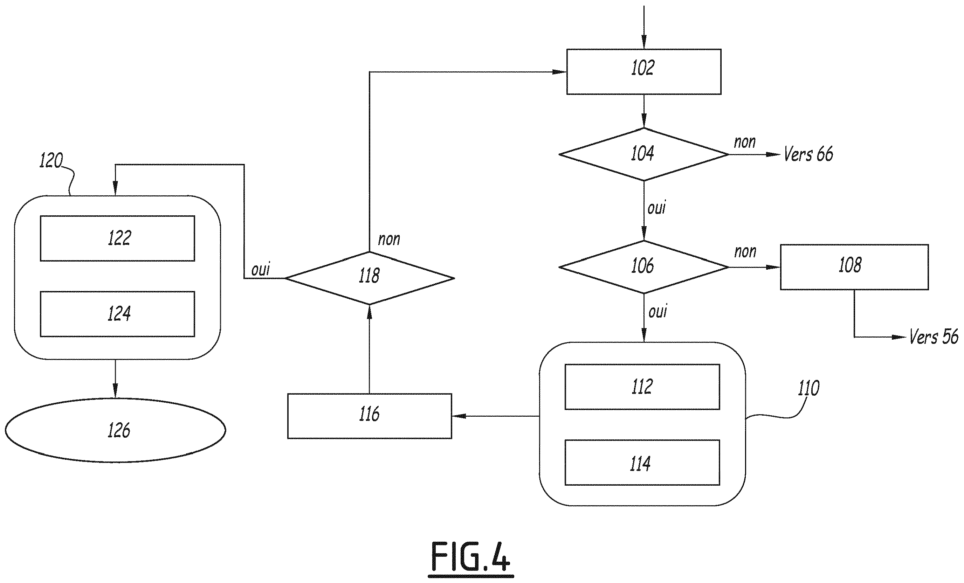

Si le résultat de la validation est positif, le procédé continue vers une phase finale 100 de détection et caractérisation dans le procédé de détection et de caractérisation de signaux faibles d'exposition à un risque, la continuation étant décrite ci-après en référence à la **figure 4.**

Cette phase finale de détection et caractérisation comprend une étape 102 de mise en oeuvre d'un module de caractérisation de signaux faibles d'exposition à un risque pour le patient, (ou signaux faibles précurseurs), qui effectue une comparaison de la signature prédictive du risque calculée, ou des signatures prédictives du risque calculées à plusieurs instants temporels à une signature de risque de référence prédéterminée.

Dans un mode de réalisation, la signature de risque de référence est une valeur de seuil, et une comparaison à la valeur de seuil est effectuée, et la détection de signaux faibles précurseurs est positive si la valeur de seuil prédéterminée est dépassée par la signature prédictive du risque calculée à au moins un instant t de l'intervalle temporel considéré. Plusieurs valeurs de seuils définissant plusieurs niveaux de risque peuvent être utilisées, ces valeurs de seuils étant préalablement mémorisées.

Dans un autre mode de réalisation, l'étape 102 met en œuvre une comparaison à une ou à des signatures de risque de référence préalablement calculées et mémorisées dans la base de données 24 de signatures de risques de référence, et la détection de signaux faibles précurseurs est positive si une distance entre signatures de risque de référence et signature prédictive du risque calculée est inférieure à un seuil de distance prédéterminé. Par exemple, chacune des signatures de risque est caractérisée par une pluralité de valeurs à des instants temporels successifs sur une durée temporelle d'évaluation des signatures de risque. Le calcul d'une distance entre signatures de risque met en œuvre dans ce cas une distance entre deux courbes, par exemple la moyenne pondérée des distances point à point.

De plus, une incertitude statistique associée à la détection est évaluée systématiquement par l'une des méthodes statistiques de l'état de l'art (loi normale ou loi de Poisson).

En cas de détection de signaux faibles précurseurs négative (réponse « non » au test 104), le procédé retourne à l'étape 66 de validation interactive par un expert.

En cas de détection de signaux faibles précurseurs positive (réponse oui au test 104), il est ensuite vérifié (test 106) s'il existe une signature de risque de référence proche de la signature prédictive du risque parmi les signatures de risque de référence préalablement mémorisées.

La proximité est évaluée en fonction d'un calcul de distance selon une mesure de distance prédéterminée. Par exemple comme indiqué ci-dessus en référence à l'étape 102, une distance entre deux courbes, respectivement une courbe représentative d'une signature de risque de référence et une courbe représentative de la signature prédictive de risque calculée, est calculée, ce qui permet de trouver la signature de risque de référence la plus proche de la signature prédictive du risque calculée. Ensuite la distance entre la signature de risque de référence la plus proche de la signature prédictive du risque calculée et la signature prédictive du risque calculée est comparée à un seuil de distance, et si elle est inférieure à ce seuil de distance, alors la réponse au test 106 est positive.

En cas, de réponse négative au test 106, une étape 108 de validation interactive par un expert est mise en oeuvre, suivie de l'étape 56 de sélection de modèles d'apprentissage par intelligence artificielle. Dans ce cas, le processus d'apprentissage est relancé avec un nouveau modèle d'apprentissage, par exemple les paramètres du modèle sont modifiés, ou un autre algorithme d'apprentissage est choisi.

En cas de réponse positive au test 106, en d'autres termes si une signature de risque de référence proche de la signature prédictive du risque a été trouvée, une étape 110 d'affichage est mise en oeuvre. Elle comporte un affichage 112 d'une simulation prédictive de l'incubation de la pathologie et une étape 114 d'affichage des caractéristiques des modèles d'IA utilisés.

Une nouvelle étape 116 de validation interactive par un expert est mise en oeuvre.

En cas de validation négative (réponse « non » au test 118), le procédé retourne à l'étape 102 de comparaison à une ou à des signatures de risque de référence.

En cas de validation positive (réponse « oui » au test 118), une étape 120 de génération de rapport est mise en oeuvre, en utilisant (étape 122) des données des bases de données préalablement mémorisées, en particulier en utilisant les scénarios de menaces associés et la cartographie de risques associée. En particulier, le scénario de menaces associé à la signature de risque de référence sélectionnée est affiché. De plus, les paramètres caractérisant le modèle de risque appliqué sont affichés, ainsi que les incertitudes probabilistes calculées.

L'expert bénéficie alors d'une information complète relative à l'évaluation du risque auquel le patient est exposé, à partir des signaux faibles observés.

En complément optionnel, un plan de propositions et de recommandations est généré (étape 124).

Ainsi, un rapport 126 est obtenu, ce rapport permettant une prise de décision clinique ou thérapeutique informée du risque détecté, suite à la détection et à la caractérisation de signaux faibles précurseurs.

Avantageusement, l'invention permet de détecter des signaux faibles d'exposition à un risque pathologique, clinique ou thérapeutique d'un patient, en lien avec une pathologie redoutée, et ainsi de conclure en ce qui concerne cette exposition à un risque de manière prédictive, avant l'apparition de signaux forts, e.g. de symptômes graves.

Avantageusement, le procédé permet de simuler l'incubation de la pathologie en fonction de signatures de risque de référence, ce qui est très utile pour une prise en charge en amont du patient.

## Revendications

**1.** Procédé de détection et de caractérisation de signaux faibles d'exposition à un risque pour un patient, un signal faible étant représentatif d'une incubation d'une pathologie, à partir de données relatives au patient collectées sur un intervalle temporel donné, le procédé étant **caractérisé en ce qu'**il comporte les étapes suivantes, mises en oeuvre par un processeur :

- à partir de données relatives au patient collectées (74) pendant l'intervalle temporel, calcul (76-80) d'une signature prédictive du risque, la signature prédictive du risque comprenant un premier terme obtenu par sommation de signatures élémentaires associées à des évènements initiateurs élémentaires, une signature élémentaire étant dépendante de paramètres comprenant une valeur de gravité de l'événement initiateur élémentaire, une fonction caractéristique de l'événement initiateur élémentaire et une fonction de pondération associée à l'événement initiateur élémentaire, au moins une partie desdits paramètres étant déterminés par mise en oeuvre d'un réseau de neurones,
- détection (102, 104) de présence d'au moins un signal faible d'exposition à un risque par comparaison de la signature prédictive du risque calculée à des signatures de risque de référence prédéterminées,
- en cas de détection positive (104), détermination (106) d'une signature prédictive de référence associée à la signature prédictive du risque calculée et caractérisation du risque associé à ladite signature de risque de référence, ladite caractérisation comportant un affichage (122) d'un scénario de menaces préalablement déterminé et enregistré en association avec ladite signature prédictive de référence.

**2.** Procédé selon la revendication 1, dans lequel la fonction de pondération associée à l'événement initiateur élémentaire est une fonction déterministe-probabiliste, dépendante d'une probabilité dudit événement initiateur élémentaire en lien avec l'incubation de ladite pathologie.

3. Procédé selon la revendication 1 ou 2, dans lequel la signature prédictive du risque comporte un deuxième terme dépendant de paires d'événements initiateurs élémentaires et d'une fonction caractéristique d'intercorrélation pour chaque paire d'événements initiateurs élémentaires.

4. Procédé selon l'une des revendications 1 à 3, dans lequel le calcul d'une signature de risque prend en compte en outre une fonction probabiliste caractéristique de bruit relatif aux données collectées.

5. Procédé selon l'une des revendications 1 à 4, dans lequel la signature élémentaire d'un événement initiateur élémentaire $E_i$ est fournie par la formule suivante :

$$2^{G_i} w_i(t) \sigma_i(t)$$

Où Gi est la gravité de l'évènement initiateur élémentaire $E_i$, $\sigma_i(t)$ est la fonction caractéristique de l'événement initiateur élémentaire $E_i$ et $w_i(t)$ est la fonction de pondération associée à l'événement initiateur élémentaire $E_i$.

6. Procédé selon l'une quelconque des revendications 1 à 5, dans lequel la signature prédictive du risque est calculée selon la formule :

$$\Gamma(t) = \left[ \sum_i 2^{G_i} w_i(t) \sigma_i(t) + \sum_{jk} \xi_{jk} w_j(t) w_k(t) 2^{G_j + G_k} \right] * B(t)$$

Où Gi est la gravité de l'évènement initiateur élémentaire $E_i$, $\sigma_i(t)$ est la fonction caractéristique de l'événement initiateur élémentaire $E_i$, $w_i(t)$ est la fonction de pondération associée à l'événement initiateur élémentaire $E_i$ ; $\xi_{jk}$ est une fonction caractéristique d'intercorrélation entre événements initiateurs élémentaires $E_j$ et $E_k$, et B(t) est une fonction probabiliste caractérisant un bruit.

7. Procédé selon la revendication 5 ou 6, dans lequel la gravité d'un évènement initiateur élémentaire prend quatre valeurs différentes représentatives respectivement d'une gravité nulle, d'une gravité mineure, d'une gravité significative ou d'une gravité sévère.

8. Procédé selon l'une quelconque des revendications 1 à 7, dans lequel l'étape de détection de présence d'au moins un signal faible d'exposition à un risque comprend en outre une évaluation statistique d'une incertitude associée à ladite détection.

9. Procédé selon l'une quelconque des revendications 1 à 8, comportant, suite à la collecte de données relatives au patient pendant l'intervalle temporel, un prétraitement desdites données collectées pour formater lesdites données collectées en données numériques, et une classification par un classifieur desdites données numériques pour obtenir des valeurs de paramètres associés aux évènements initiateurs élémentaires.

10. Procédé selon l'une quelconque des revendications 1 à 9, comportant une phase d'initialisation (50) d'une base de données (24) des signatures de risque de référence, en lien avec un périmètre pathologique défini, en fonction de données de santé de cohortes de patients et de validations d'experts, et une mémorisation des signatures de risque de référence, de scénarios de menaces associés et d'une cartographie de risques associée.

11. Programme d'ordinateur comportant des instructions logicielles qui, lorsqu'elles sont exécutées par un dispositif programmable, mettent en oeuvre un procédé de détection et de caractérisation de signaux faibles d'exposition à un risque pour un patient conforme aux revendications 1 à 10.

12. Système de détection et de caractérisation de signaux faibles d'exposition à un risque pour un patient, un signal faible étant représentatif d'une incubation d'une pathologie, à partir de données relatives au patient collectées sur un intervalle temporel donné, le système étant **caractérisé en ce qu'**il comporte au moins un système de calcul, comportant un processeur configuré pour mettre en œuvre :

- à partir de données relatives au patient collectées pendant l'intervalle temporel, un module (36) de calcul d'une signature prédictive du risque, la signature prédictive du risque comprenant un premier terme obtenu par sommation de signatures élémentaires associées à des évènements initiateurs élémentaires, une signature élémentaire étant dépendante de paramètres comprenant une valeur de gravité de l'événement initiateur élémentaire, une fonction caractéristique de l'événement initiateur élémentaire et une fonction de pondération associée à l'événement initiateur élémentaire, au moins une partie desdits paramètres étant déterminés par mise en oeuvre d'un réseau de neurones,
- un module (38) de détection de présence d'au moins un signal faible d'exposition à un risque par comparaison de la signature prédictive du risque calculée à des signatures de risque de référence prédéterminées,
- en cas de détection positive, application d'un module (40) de détermination d'une signature prédictive de référence associée à la signature prédictive du risque calculée et de caractérisation du risque associé à la signature de risque de référence, comportant un module affichage d'un scénario de menaces préalablement déterminé et enregistré en association avec ladite signature prédictive de référence.

FIG.1

FIG.2

FIG.3

FIG.4

Europäisches
Patentamt

European
Patent Office

Office européen
des brevets

# RAPPORT DE RECHERCHE EUROPEENNE

Numéro de la demande

**EP 22 20 8493**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 11 056 242 B1 (JAIN PRADUMAN [US] ET AL) 6 juillet 2021 (2021-07-06)<br>* figures 1-23 *<br>* colonne 1, ligne 1 – colonne 22, ligne 23 *<br>* colonne 27, ligne 36 – colonne 32, ligne 59 *<br>* colonne 69, ligne 20 – colonne 73, ligne 59 *<br>* colonne 121, ligne 60 – colonne 129, ligne 63 *<br>----- | 1-12 | INV.<br>G16H50/20<br>G16H50/30<br><br>ADD.<br>G16H10/60<br>G16H50/70 |
| A | Anonymous: "Signal faible – Wikipédia", , 5 novembre 2021 (2021-11-05), XP055968518, Extrait de l'Internet: URL:https://fr.wikipedia.org/w/index.php?title=Signal_faible&oldid=187741910 [extrait le 2022-10-06]<br>* le document en entier *<br>----- | 1-12 | |

**DOMAINES TECHNIQUES RECHERCHES (IPC)**

G16H

Le présent rapport a été établi pour toutes les revendications

| Lieu de la recherche | Date d'achèvement de la recherche | Examinateur |
|---|---|---|
| Munich | 21 mars 2023 | Sasa Bastinos, Ana |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.........................................................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 22 20 8493

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

21-03-2023

| Document brevet cité au rapport de recherche | | Date de publication | Membre(s) de la famille de brevet(s) | | Date de publication |
|---|---|---|---|---|---|
| US 11056242 | B1 | 06-07-2021 | US | 11056242 B1 | 06-07-2021 |
| | | | US | 11302448 B1 | 12-04-2022 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3009615

**Littérature non-brevet citée dans la description**

- **C.BOMBARDIER et al.** Dérivation of SLEDAI : a disease activity index for lupus patients. *Arthritis Rheum,* 1992, vol. 35, 630-640